Europäisches Patentamt

European Patent Office

Office européen des brevets

(11) Publication number: **0 234 372**
**A2**

(12) **EUROPEAN PATENT APPLICATION**

(21) Application number: **87101682.0**

(22) Date of filing: **07.02.87**

(51) Int. Cl.³: **A 61 F 5/02**

(30) Priority: **17.02.86 IT 1942686**

(43) Date of publication of application:
**02.09.87 Bulletin 87/36**

(84) Designated Contracting States:
**CH DE ES FR GB IT LI**

(71) Applicant: **Bianchini, Sergio**
**Via Alpi Giulie 39**
**I-28044 Verbania Intra (Province of Novara)(IT)**

(72) Inventor: **Bianchini, Sergio**
**Via Alpi Giulie 39**
**I-28044 Verbania Intra (Province of Novara)(IT)**

(74) Representative: **Modiano, Guido et al,**
**MODIANO, JOSIF, PISANTY & STAUB Modiano &**
**Associati Via Meravigli, 16**
**I-20123 Milan(IT)**

(54) **Corset for the treatment of scoliosis in vertebral pathology.**

(57) The present invention relates to a corset for the treatment of scoliosis in vertebral pathology, of the kind comprising a pair of pelvic valves (2a, 2b) which support a front rod (3) and at least one rear rod (4), and at least one thrust hand (9, 10) associated with a supporting element connected to at least one of the front rods (3) or rear rods (4). The corset (1a) according to the present invention has the peculiarity that it comprises at least one portion, which includes the thrust hand (9,10), movable with respect to the remaining part of the corset (1a) in order to allow the patient a controlled movement within the corset (1).

Fig.1

EP 0 234 372 A2

CORSET FOR THE TREATMENT OF SCOLIOSIS IN VERTEBRAL PATHOLOGY

The present invention relates to a corset for the treatment of scoliosis in vertebral pathology.

In the orthopaedic field, in order to treat scoliosis, a number of corsets have long been known which are applied to the patient for preset lengths of time, according to the extent of the deformation of the spinal column, in order to correct the deformities, gradually making the configuration of the spinal column as close as possible to the normal configuration.

The currently best-known types of corset are: the Milwaukee corset and the Lyonese corset.

The Milwaukee corset is substantially composed of a pair of elements or pelvic valves which support two rigid rear rods and a rigid front rod, adjustable in height, which support, at their upper end, a cervical ring provided with a sub-mandibular rest and with two suboccipital rests. Lateral thrust hands, or pressers, are applied to the sides of the corset, to specifically correct the deformities.

The Lyonese corset is substantially composed of a pair of pelvic valves, which support a front rod and a rear rod, and of at least one thrust hand which is associated to a supporting element fixed to at least one of the front or rear rods. Underarm plates or supports are furthermore provided, contributing to the general balance of the thrusts effected by the corset.

Such known types of corset, though they achieve an effective correction of costal and vertebral deformities, still have some disadvantages.

The main one is due to the absolute rigidity of the corset after it has been adjusted and closed around the patient. This rigidity prevents most motions of the spinal column and can therefore cause its progressive stiffening, forcing the patient to undergo long sessions of rehabilitation gymnastics during and after the corrective treatment.

Another disadvantage is found in performing gymnastic exercises which assist the treatment with the corset; these exercises are performed by the patient while wearing the corset, and some types of motion practically cancel the preset thrust exerted by the thrust hands on the deformity to be corrected.

The substantially absolute rigidity of the corset furthermore gives rise to considerable disadvantages regarding its tolerability on the part of the patient.

The aim proposed by the present invention is to eliminate the above described disadvantages by providing a corset for the treatment of scoliosis, which allows controlled movements of the patient inside the corset, which movements can be used for effective corrective gymnastics to assist the action of the corset and to give relief to the patient, yet maintaining the corrective effects widely confirmed in the known types.

Within the scope of this aim, an object of the invention is to provide a corset which, during the movements performed in the corrective gymnastics sessions, maintains or increases controllably the thrusts which act on the deformities even for those movements which naturally reduce the extent of the deformities.

A not least object of the present invention is to propose a corset whose structure maintains the positive and fundamental characteristics of known corsets in order to preserve their effectiveness, as well as their providability now established in the known types by years of research and of studies in this field.

This aim, as well as this and other objects which will become apparent hereinafter, are achieved by a corset for the treatment of scoliosis in vertebral pathology, comprising: a pair of pelvic valves which support a front rod and at least one rear rod, and at least one thrust hand associated with a supporting element connected to at least one of said front rods or rear rods, characterized in that it comprises at least one portion, which includes said at least one thrust hand, movable with respect to the remaining part of the corset in order to allow the patient a controlled movement within the corset.

Further characteristics and advantages of the invention will become apparent from the description of two preferred, but not exclusive, embodiments of the corset according to the invention, illustrated only by way of non-limitative example in the accompanying drawings, where:

Fig. 1 is is a perspective view of the corset according to the invention in the first embodiment;

Figs. 2 and 3 are lateral elevation views of the corset of Fig. 1, respectively in rigid position and with the upper part partially rotated with respect to the lower part;

Fig. 4 is an enlarged perspective view of a detail of the corset illustrated in the preceding figures;

Fig.5 is a perspective view of the corset according to the invention in the second embodiment;

Fig. 6 is a lateral elevation view of the corset of fig. 5;

Figs. 7 and 8 are schematic top plan views of the corset in the second embodiment, illustrating the movements of the movable portion according to the invention.

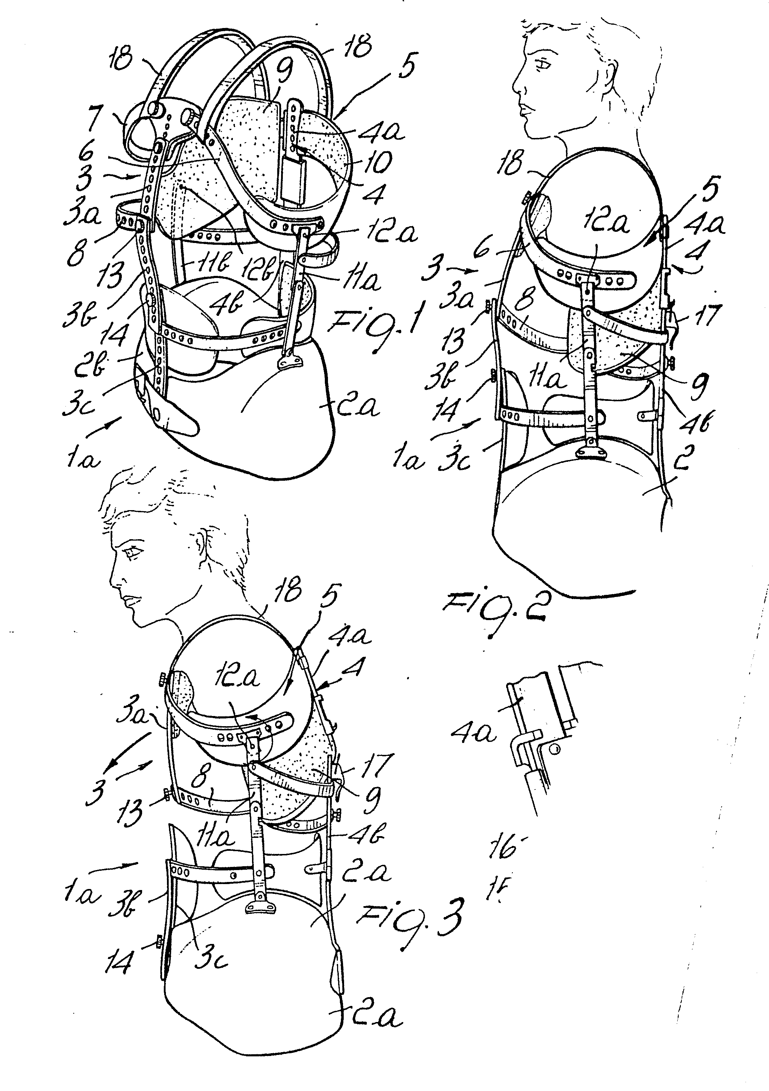

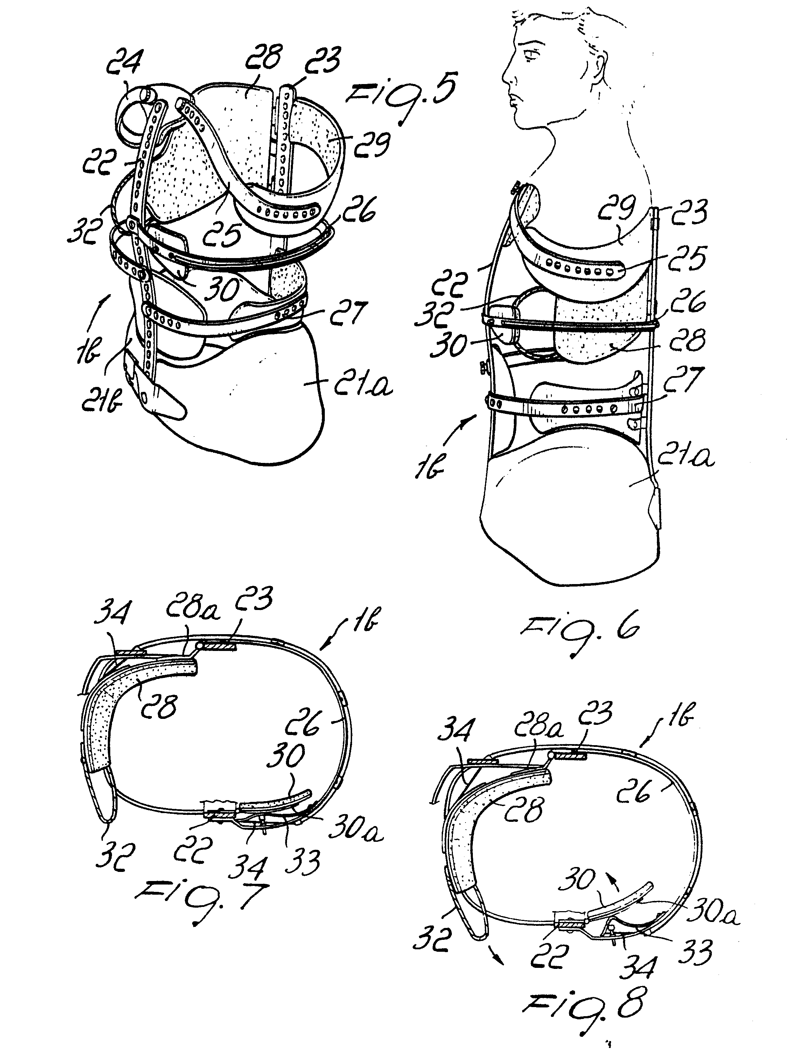

With reference to the above described figures, the corset according to the invention in the first embodiment, generally indicated with the reference numeral 1a, comprises, similarly to known corsets, a pair of pelvic valves 2a and 2b which support a front rod 3 and a rear rod 4 in rigid material. One or more supporting elements, for one or more thrust hands which are arranged on the deformities to be corrected, are associated to the vertical rods.

According to the invention, the corset is provided with a portion which comprises at least one thrust hand and is movable with respect to the remaining part of the corset.

In this first embodiment, the movable portion according to the invention is composed of a frame, generally indicated with the reference numeral 5, which comprises the upper part 3a of the front rod, the upper part of the rear rod 4a and transverse rods 6, 7 and 8 which support the thrust hand 9, which in this case also acts as right underarm plate and the left underarm plate 10. The connection between the frame 5 and the remaining part of the corset is achieved so that the frame 5 can oscillate, with a back-and-forth motion, around an axis which is transverse with respect to the longitudinal axis of the corset and parallel to an ideal front plane of the corset.

For this purpose, a pair of lateral rods 11a and 11b is provided, which extend on opposite sides of the pair of pelvic valves towards the frame 5 and are connected thereto by means of a pair of hinges 12a and 12b which indeed define, for the frame 5, an axis of oscillation which is transverse with respect to the longitudinal axis of the corset and is parallel to its front plane.

The axis of oscillation can be, according to the requirements, perpendicular or tilted, by a preset angle, with respect to the longitudinal axis of the corset.

It may be furthermore arranged that the hinges 12a and 12b be movable along the lateral rods 11a and 11b to alter the oscillation of the frame 5 with respect to the remaining part of the corset.

The lateral rods 11a and 11b may extend on a same plane, parallel to the front plane of the corset, or on two different planes, again parallel to the front plane of the corset.

In order to allow the frame 5 to perform the oscillating motion described above, the front rod and the rear rod are provided, in an intermediate portion of their length, with an interruption and, at said interruption, controllably operatable means are provided for the rigid coupling of the frame 5 to the remaining part of the corset.

More in detail, the intermediate part of the front rod is composed of a section of rigid rod 3b which has, depending on whether it is desired to lock or release the frame 5, the possibility of being fixed with its ends, e.g. by means of screws 13 and 14, to the upper part 3a of the front rod or to its lower part 3c which is fixed to the pelvic valves, or of being removed so that the lower end of the upper part 3a is spaced from the lower part 3c to allow the back-and-forth motion of the frame 5. In particular, the rigid section of rod 3b may be rotated around its lower end and superimposed on the lower part 3c of the front rod.

The controllably operatable means for the rigid coupling of the frame 5 to the remaining part of the corset comprise, besides the rigid section of rod 3b, a coupling device 15 arranged at the interruption in the rear rod.

The coupling device 15 comprises a fixed hook 16 rigidly associated to the upper part 4a of the rod and a lever 17 which is hinged to the lower part 4b of the rear rod and can firmly engage the hook 16.

Means may furthermore be fixed to the frame 5 to associate the frame with the shoulders of the patient; in the illustrated case, such means are composed of straps 18 which are fixed to the frame 5 and which are passed over the shoulders of the patient.

The corset in the second embodiment, generally indicated with the reference numeral 1b, comprises a pair of pelvic valves 21a and 21b which support, similarly to the first embodiment, a front rod 22 and a rear rod 23 to which one or more thrust hands are associated by supporting means.

More in detail, transverse rods 24, 25, 26 and 27 are provided, which are associated to the front rod or to the rear rod, or to both, to stiffen the corset and to support at least one thrust hand 28, which also acts as a right underarm plate, as well as the left underarm plate 29 and a second thrust hand 30.

The movable portion according to the invention comprises at least the thrust hand 28 with its supporting element 28a, and, in the illustrated case, also the second thrust hand 30. The thrust hand 28 is hinged with one end of the related supporting element 28a to the rear rod 23 and can oscillate around an axis which is substantially parallel to the longitudinal axis of the corset to approach, and respectively move away from, the longitudinal axis of the corset.

Thus, similarly, the second thrust hand 30, which is arranged substantially in a region diametrally opposite with respect to the thrust hand 28, is hinged with one end of its supporting element 30a to the front rod and can oscillate around an axis which is substantially parallel to the longitudinal axis of the corset to approach, and respectively move away from, the longitudinal axis of the corset with its free end.

For the motion of the thrust hand 28, first means for oscillating are provided, substantially composed of a connecting bracket or stirrup 32, associated with the thrust hand 30, which can be hooked by the patient with one hand and pulled forward.

Second means for oscillating are furthermore provided, which act on the second thrust hand 30 to achieve its motion. The second means for oscillating, in the case illustrated, are composed of a flat spring 33 interposed between the transverse rod 26 and the second thrust hand. An end of the flat spring 33 is fixed to the transverse rod 26, and the other end is connected to a tension wire 34 to achieve, by tensioning the wire, a flexing of the flat spring which pushes the second thrust hand toward the inside of the corset.

The same effect could be achieved with an eccentric, supported by the transverse rod 26 and acting on the second thrust hand or with another technically equivalent means.

Advantageously, the first means for oscillating and the second means for oscillating are connected to each other to simultaneously achieve on opposite sides the motion towards the longitudinal axis of the corset of the thrust hand 28 and of the second thrust hand 30.

This connection may be provided simply by associating the tensioning wire 34 to the thrust hand 28.

After what has been described, the use of the corset according to the invention is as follows.

With the corset closed around the patient, it is possible to achieve a controlled movement of the patient within the corset during his normal activity or during sessions of corrective gymnastics.

More precisely, with a corset provided as in the first embodiment 1a, it is possible to achieve a back-and-forth flexing of the trunk of the patient if the coupling device 15, provided on the rear rod 4, is uncoupled and the rigid section of rod 3b, arranged at the front, is removed. In this manner, the frame 5 can perform a limited oscillation, yet maintain pressure on the deformities of the patient.

When it is desired to restore the rigidity of the corset, one proceeds by coupling the frame 5 back to the remaining part of the corset.

In the second embodiment, the corset 1b allows a torsion of the trunk of the patient within the corset. In this case, the patient grasps with his hand the coupling bracket 32 and pulls it forwards; in this manner, there is a simultaneous approach of the free ends of the thrust hands 28 and 30 to the longitudinal axis of the corset which has the effect of aiding in the torsion of the trunk of the patient.

By releasing the coupling bracket 32, the thrust hands return in idle position by virtue of the presence of the patient in the corset.

In practice, it has been found that the corset according to the invention fully achieves the intended aim, allowing the patient to perform controlled movements within the corset which can be used for more effective corrective gymnastics and to relieve the discomfort due to the rigidity of the corset.

In particular, also for those movements of the patient which lead to a reduction of the deformities, the corset according to the invention maintains a thrust action on the deformities which would not be possible in the completely rigid corsets of the known type.

The corset thus conceived is susceptible to numerous modifications and variations, all of which are within the scope of the inventive concept; moreover, all the details may be replaced with technically equivalent elements.

In practice, the materials employed, as well as the dimensions, may be any according to the requirements and the state of the art.

## CLAIMS

1. Corset for the treatment of scoliosis in vertebral pathology, comprising: a pair of pelvic valves (2a, 2b, 21a, 21b) which support a front rod (3, 22) and at least one rear rod (4, 23), and at least one thrust hand (9) associated with a supporting element connected to at least one of said front rods (3, 22) or rear rods (4, 23), characterized in that it comprises at least one portion, which includes said at least one thrust hand (9, 28), movable with respect to the remaining part of the corset (1a, 1b) in order to allow a controlled movement of the patient within the corset (1a, 1b).

2. Corset according to claim 1, characterized in that said movable portion comprises a frame (5) composed of the upper part (3a) of said front rod (3), of the upper part (4a) of said rear rod (4) and of transverse rods (7,8) supporting said at least one thrust hand (9), said front rod (3) and said rear rod (4) being provided, in an intermediate section of their length, with an interruption, and said frame (5) being connected, so as to be able to oscillate around an axis of oscillation which is transverse to the longitudinal axis of the corset and parallel to an ideal front plane of the corset, to the remaining part of the corset (1a) for a back-and-forth flexing of the patient within the corset (1a).

3. Corset according to claims 1 and 2, characterized in that it comprises a pair of lateral rods (11a, 11b) extending from opposite parts of said pair of pelvic valves (2a, 2b) and supporting said frame (5), said lateral rods (11a, 11b) being connected to said frame (5) by means of a pair of hinges (12a, 12b) which define said axis of oscillation which is transverse with respect to the longitudinal axis of the corset (1a).

4. Corset, according to claim 3, characterized in that said hinges (12a, 12b) are movable along said lateral rods (11a, 11b).

5. Corset, according to claims 1 and 2, characterized in that it comprises controllably operatable means (15, 3b) for the rigid coupling of said frame (5) to the remaining part of the corset (1a).

6. Corset, according to claim 5, characterized in that said controllably operatable means comprise a coupling device (15) arranged at the interruption of said rear rod (4).

7. Corset, according to claim 5, characterized in that said controllably operatable means comprise a rigid segment of rod (3b) associable with its ends respectively with said frame (5) and with the lower part (3c) of said front rod associated to said pair of pelvic valves (2a, 2b) on the front side, said rigid section of rod (3b) being removable and said upper part of said front rod (3a) being spaced from the lower part (3c) associated with said pair of pelvic valves (2a, 2b).

8. Corset, according to claims 1 and 2, characterized in that it comprises means (18) for associating said frame to the shoulders of the patient.

9. Corset, according to claim 1, characterized in that said movable portion is composed of said supporting element (28a) and of at least one thrust hand (28), first means (32) being provided for the oscillation of said at least one thrust hand towards and away from the longitudinal axis of the corset (1b) for the torsion of the patient within the corset (1b).

10. Corset, according to claims 1 and 9, characterized in that it comprises a second thrust hand (30) arranged substantially diametrally opposite to said at least one thrust hand (28), second means (33) being provided for the oscillation of said second thrust hand, towards and away from the longitudinal axis of the corset (1b).

11. Corset, according to claims 9 and 10, characterized in that said first and said second means (33) for the oscillation of said at least one thrust hand (28) and of said second thrust hand (30) are operatively connected to each other for a simultaneous approach, on opposite sides, to the longitudinal axis of the corset (1b) of said at least one thrust hand (28) and of said second thrust hand (30).

12. Corset, according to claims 9 and 10, characterized in that said first means (32) for the oscillation of said at least one thrust hand comprise an engagement bracket (32) which can be operated by the patient to move said at least one thrust hand (28) closer to the longitudinal axis of the corset (1b).

13. Corset, according to claims 9 and 10, characterized in that said supporting element (28a) associated with said at least one thrust hand (28) and a second supporting element (30a) associated to said said second thrust hand (30) are hinged to the remaining part of the corset (1b) with an axis of oscillation which is substantially parallel to the longitudinal axis of the corset (1b).

1/2    0234372

Fig.1

Fig.2

Fig.3

Fig.5

Fig.6

Fig.7

Fig.8